# EUROPEAN PATENT APPLICATION

(11) **EP 4 606 895 A1**
(43) Date of publication of application: **27.08.2025**
(21) Application number: 23878938.2
(22) Date of filing: 27.09.2023
(51) Int. Cl.: C12N 15/10, C12N 15/62, C40B 50/14, C40B 40/08

(54) **MULTI-OMICS LIBRARY CONSTRUCTION METHOD, DETECTION METHOD, AND RELATED KIT**

(30) Priority: 21.10.2022 CN 202211296854
(71) Applicant: BGI Shenzhen, Shenzhen, Guangdong 518083 (CN); BGI Shenzhen Co., Ltd, Shenzhen, Guangdong 518083 (CN)
(72) Inventor: HENG, Yang, Shenzhen, Guangdong 518083 (CN); LIAO, Sha, Shenzhen, Guangdong 518083 (CN); CHEN, Ao, Shenzhen, Guangdong 518083 (CN); XU, Xun, Shenzhen, Guangdong 518083 (CN); ZHANG, Wenwei, Shenzhen, Guangdong 518083 (CN)
(74) Representative: Novagraaf Technologies
(86) International application number: PCT/CN2023/121833
(87) International publication number: WO 2024/082945

(57) **Abstract**

A multi-omics library construction method, a detection method, and a related kit. The multi-omics library construction method includes: placing a sectioned sample on a chip having a PolyA capture function for protein capture and mRNA capture, and performing protein capture using a nucleic acid-conjugated antibody; performing reverse transcription on captured mRNA and complementary strand synthesis on a nucleic acid sequence on the nucleic acid-conjugated antibody to respectively obtain cDNA and a complementary strand of the nucleic acid sequence; and performing library construction on the cDNA and the complementary strand of the nucleic acid sequence, thereby obtaining a transcriptome library and a proteome library of the sectioned sample. With an antibody-conjugated nucleic acid having PolyA, the antibody-conjugated nucleic acid PolyA bound to an antigen protein and mRNA are captured at the same time through a PolyT structure on a chip, such that simultaneous construction of a proteome library and a transcriptome library and subsequent co-localization detection are implemented, thereby realizing full-tissue field-of-view detection of a transcriptome and a proteome.

## Description

### Technical Field

The present invention relates to the technical field of multi-omics detection, and specifically, to a multi-omics library construction method, a detection method, and a related kit.

### Background

Common technologies used to detect proteins include Immunofluorescence (IF) and immunohistochemistry. The IF is a gold standard for protein detection. However, due to the limitations of fluorescent channels and antibody species, usually the expression of only 1-5 proteins can be detected in one tissue section. Mature products on the market that can detect the expression of multiple proteins cannot simultaneously detect the expression of a transcriptome. For example, a product of Olink Proteomics can only detect protein expression in a liquid phase (blood or body fluid), but cannot detect protein localization and expression in the tissue section. The products of AkoyaBiosciences cannot detect the transcriptomic expression although they may detect the protein expression on the tissue section. A GeoMx Digital Spatial Profiler technology of NanoString may detect the transcriptome and may also detect the protein expression. However, the detection field of view of the product is limited, and when a region of interest for a tissue is circled, the product can usually only detect dozens of regions with a diameter of 50-100 µm in size, but cannot perform full field-of-view detection on the section tissue. Although a Spatial Gene Expression product of 10×Genomics may simultaneously detect the expression of a whole transcriptome and a proteome in a field of view of 0.65 cm×0.65 cm, the resolution of the product is too low, and one Spot is about 55 µm, which does not reach single-cell resolution of detection.

Therefore, in terms of simultaneous detection of the proteome and the transcriptome, there is room for improvement in the related art.

### Summary

The present invention is mainly intended to provide a multi-omics library construction method, a detection method, and a related kit, so as to provide a new solution that can realize full-tissue field-of-view detection of a transcriptome and a proteome.

In order to implement the above objective, an aspect of the present invention provides a multi-omics library construction method. The method comprises: S1, a sectioned sample is placed on a chip having a Poly A capture function for protein capture and mRNA capture, wherein the protein capture is performed with a nucleic acid-conjugated antibody, the nucleic acid-conjugated antibody comprises an antibody and a nucleic acid sequence conjugated thereto, the nucleic acid sequence successively comprises in a direction away from the antibody: a PCR adapter region, an antibody barcode region, and a Poly A sequence, the chip comprises Poly T, the nucleic acid-conjugated antibody binds to the Poly T on the chip through the Poly A sequence to perform antibody-bound antigen protein capture, and mRNA in the sectioned sample binds to the Poly T on the chip through the Poly A of the mRNA to perform mRNA capture; S2, reverse transcription on the captured mRNA and complementary strand synthesis on the nucleic acid sequence of the antigen-bound nucleic acid-conjugated antibody to respectively obtain cDNA and a complementary strand of the nucleic acid sequence on the nucleic acid-conjugated antibody; and S3, library construction is performed on the cDNA and the complementary strand of the nucleic acid sequence of the nucleic acid-conjugated antibody to obtain a transcriptome library and a proteome library of the sectioned sample.

Further, S1 comprises: S11, blocking the chip using a blocking solution to obtain a blocked chip; S12, incubating the nucleic acid-conjugated antibody with the blocked chip to obtain a protein capture chip; and S13, performing a permeabilization treatment on the protein capture chip to capture the mRNA and the nucleic acid sequence, thereby obtaining a permeabilized chip.

Further, S2 comprises: performing reverse transcription on the captured mRNA and complementary strand synthesis on the nucleic acid sequence on the permeabilized chip, thereby obtaining a synthesized product, wherein the synthesized product comprises the cDNA and the complementary strand of the nucleic acid sequence; and preferably, a temperature for the reverse transcription is 40 °C-44 °C, and a time is 1.5 hours-3 hours.

Further, S3 comprises: S31, performing tissue removal and probe digestion successively on the sectioned sample after reverse transcription and complementary strand synthesis on the permeabilized chip, thereby obtaining a digestion solution containing the synthesized product; and S32, separating and purifying the synthesized product from the digestion solution, and respectively obtaining the proteome library and the transcriptome library through amplification.

Further, the blocking solution comprises 3×SSC, 5%-10%(v/v) of serum, 0.1%(v/v) of Triton-x-100, 0.1 mg/mL-1 mg/mL of salmon sperm DNA, 0.5 µM-10 µM of 22 bp-32 bp PolyA, and 5%(v/v) of a RNase inhibitor; preferably, a concentration of the 22 bp-32 bp PolyA in the blocking solution is 1 µM-10 µM, more preferably 5 µM-10 µM, and further preferably 8 µM-10 µM; and preferably, a concentration of the salmon sperm DNA in the blocking solution is 0.5 mg/mL-1 mg/mL.

Further, in S12, before incubating the nucleic acid-conjugated antibody with the blocked chip, the method further comprises a step of diluting the nucleic acid-conjugated antibody with an antibody diluent. The antibody diluent is any one selected from the group consisting of a blocking solution, a PBS buffer containing PolyA and DNA, or 2-3×SSC buffer containing PolyA and DNA. In the PBS buffer and the SSC buffer, a concentration of the PolyA is 0.5 µM-10 µM, a concentration of the DNA is 0.1 mg/ml-1 mg/ml, and preferably, the DNA is salmon sperm DNA.

Further, S12 comprises: incubating the nucleic acid-conjugated antibody with the blocked chip to bind the nucleic acid-conjugated antibody to an antigen, thereby obtaining an antibody-antigen complex; and the antibody-antigen complex is placed at 60 °C-70 °C, preferably 65 °C-70 °C, to react in 0.1×SSC buffer for 5-15 min, preferably 10 min, thereby obtaining the protein capture chip.

Further, a temperature for the permeabilization treatment is 35 °C-38 °C, and a time for the permeabilization treatment is 3 min-20 min.

Further, a temperature for the tissue removal is 50 °C-60 °C, and a time for the tissue removal is 20-40 min; and preferably, a temperature for the digestion is 50 °C-60 °C, and a time for the digestion is 2.5 hours-4 hours.

Further, the Poly T is arranged as a plurality of clusters on the chip, and a distance between two adjacent clusters is 495 nm-505 nm; preferably, in the nucleic acid-conjugated antibody, a length of the PCR adapter region is 18 bp-23 bp, a length of the antibody barcode region is 8 bp-20 bp, and a length of the PolyA is 22 bp-35 bp; and preferably, the antibody portion of the nucleic acid-conjugated antibody is one or more antibodies selected from the group consisting of a CD169 antibody, a CD3 antibody, a CD8 antibody, a CD19 antibody, a CD20 antibody, a CD21 antibody, a CD45R-B220 antibody, a CD163 antibody, a CD38 antibody, a CD11b antibody, a CD140a antibody, a CD335 antibody, a CD371 antibody, a CD90.2 antibody, a TER-119 antibody, a CD274 antibody, a CD279 antibody, a CD56 antibody, a CD14 antibody, a CD340 antibody, a CD324 antibody, a CD38 antibody, a CD29 antibody, a CD68 antibody, a CD44 antibody, a CD21/35 antibody, an IgD antibody, a CD5 antibody, a CD4 antibody, an IgM antibody, a CD79b antibody, a CD11c antibody, an F4/80 antibody, a CD27 antibody, a CD31 antibody, or a CD8a antibody.

A second aspect of the present application provides a multi-omics detection method. The method comprises: constructing a proteome library and a transcriptome library of a sectioned sample to be detected with any one of the above multi-omics library construction methods; and performing sequencing on the proteome library and the transcriptome library to obtain proteome information and transcriptome information of the sectioned sample.

A third aspect of the present application provides a kit for constructing multi-omics libraries. The kit comprises any of the following: a Poly A capture chip, a nucleic acid-conjugated antibody, a blocking solution, a tissue permeabilization solution, a tissue removal solution, and a digestion solution, the blocking solution comprises 3×SSC, 5%-10%(v/v) of serum, 0.1%(v/v) of Triton-x-100, 0.1 mg/mL-1 mg/mL of salmon sperm DNA, 0.5 µM-10 µM of 22 bp-32 bp PolyA, and 5%(v/v) of a RNase inhibitor.

Further, a concentration of the 22 bp-32 bp PolyA in the blocking solution is 1 µM-10 µM, more preferably 5 µM-10 µM, and further preferably 8 µM-10 µM; preferably, a concentration of the salmon sperm DNA in the blocking solution is 0.5 mg/mL-1 mg/mL; preferably, the Poly A capture chip refers to a capture chip containing Poly **T,** the nucleic acid-conjugated antibody comprises an antibody and a nucleic acid sequence conjugated to the antibody, and the nucleic acid sequence successively comprises in a direction away from the antibody: a PCR adapter region, an antibody barcode region, and a Poly A sequence; preferably, the Poly T is arranged as a plurality of clusters on the Poly A capture chip, and a distance between two adjacent clusters is 495 nm-505 nm; and preferably, in the nucleic acid-conjugated antibody, a length of the PCR adapter region is 18-23 bp, a length of the antibody barcode region is 8-20 bp, and a length of the PolyA is 22 bp-35 bp.

Through the application of technical solution of the present invention, applying an antibody-conjugated nucleic acid having PolyA, an antibody recognition sequence, and a library amplification sequence, the antibody-conjugated nucleic acid PolyA bound to an antigen protein and transcriptome PolyA are captured at the same time through the PolyT structure on the chip, such that simultaneous construction of the proteome library and the transcriptome library, as well as subsequent co-localization detection of the proteome and transcriptome, are implemented.

### Brief Description of the Drawings

The drawings, which form a part of the present application, are used to provide a further understanding of the present invention. The exemplary embodiments of the present invention and the description thereof are used to explain the present invention, but do not constitute improper limitations to the present invention. In the drawings:
Fig. 1 is a schematic structural diagram of a nucleic acid-conjugated antibody used in preferred embodiments of the present application.
Fig. 2 is a schematic diagram of multi-omics library construction according to preferred embodiments of the present application.
Fig. 3 is a diagram of multi-omics detection results according to Embodiment I of the present application.
Fig. 4 is a diagram of multi-omics detection results according to Embodiment II of the present application.
Fig. 5 is a diagram of multi-omics detection results according to Embodiment III of the present application.
Fig. 6 is a diagram of multi-omics detection results according to Embodiment IV of the present application.
Fig. 7 is a diagram of multi-omics detection results according to Embodiment V of the present application, wherein S stands for signal, N stands for noise, S/N stands for signal to noise ratio.
Fig. 8 is a diagram of multi-omics detection results according to Embodiment VI of the present application, wherein S stands for signal, N stands for noise, S/N stands for signal to noise ratio.
Fig. 9 is a diagram of multi-omics detection results according to Embodiment VII of the present application.
Fig. 10 is a diagram of multi-omics detection results according to Embodiment VIII of the present application.
Fig. 11 is a diagram of multi-omics detection results according to Embodiment IX of the present application.
Fig. 12 is a diagram of multi-omics detection results according to Embodiment X of the present application.
Fig. 13 is a diagram of multi-omics detection results according to Embodiment XI of the present application.
Fig. 14 is a diagram of multi-omics detection results according to Embodiment XII of the present application.

### Detailed Description of the Embodiments

It is to be noted that the embodiments in the present application and the features in the embodiments may be combined with one another without conflict. The present invention will be described below in detail with reference to the embodiments.

As mentioned in the Background, there is room for improvement in the prior art in terms of solutions for detecting both a transcriptome and a proteome in a full-tissue field of view. In order to improve this situation, the inventors of the present application attempted to realize the simultaneous capture of the Poly A in an antibody-conjugated nucleic acid bound to an antigen protein and transcriptome PolyA with an antibody-conjugated nucleic acid method based on a self-developed spatiotemporal omics technology and using a PolyT structure on a chip, such that simultaneous construction of a proteome library and a transcriptome library, as well as co-localization detection of the proteome and transcriptome, are implemented. Based on this, the applicant proposes a series of protection solutions of the present application.

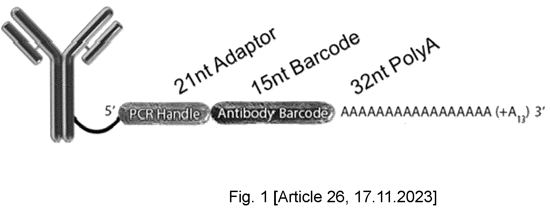

A typical implementation of the present application provides a multi-omics library construction method. **The** method comprises: S1, a sectioned sample is placed on a chip having a Poly A capture function for protein capture and mRNA capture, where the protein capture is performed by using a nucleic acid-conjugated antibody, as shown in Fig. 1, the nucleic acid-conjugated antibody comprises an antibody and a nucleic acid sequence conjugated thereto, the nucleic acid sequence successively comprises according to a direction away from the antibody: a PCR adapter region (which may be, for example, 21 bp), an antibody barcode region (which may be, for example, 15 bp), and a Poly A (which may be, for example, 32 bp) sequence, the chip carries Poly **T,** the nucleic acid-conjugated antibody binds to the Poly T on the chip through the Poly A (32 bp) sequence to capture an antibody-bound antigen protein, and mRNA in the sectioned sample binds to the Poly T on the chip through the Poly A for capture; S2, reverse transcription on the captured mRNA and complementary strand synthesis on the nucleic acid sequence of the protein-bound nucleic acid-conjugated antibody, to respectively obtain a cDNA sequence and a complementary strand of the nucleic acid sequence on the nucleic acid-conjugated antibody; and S3, library construction is performed on the cDNA sequence and the complementary strand of the nucleic acid sequence of the nucleic acid-conjugated antibody to obtain a proteome library and a transcriptome library of the sectioned sample.

In the present application, a spatiotemporal proteome is implemented by using a type of nucleic acid-coupled antibodies (commercial products may be used, for example, Totalseq-Antibody series sold by Biolegend). This type of antibody is a normal antibody conjugated to a section of the nucleic acid sequence. **The** nucleic acid sequence is constituted by three portions, which respectively are the PCR adapter region (configured to be used for subsequent library amplification, and according to different antibodies, a specific length is different, which may be, for example, 21 bp), the antibody barcode region (configured to recognize the difference in the antibodies, and a specific length may also be rationally set, which may be, for example, 15 bp), and the PolyA (configured to bind to the Poly T fixed at a specific position of the chip, and a specific length may also be properly adjusted, which may be, for example, 32 bp) sequence.

According to the multi-omics library construction method of the present application, applying an antibody-conjugated nucleic acid having PolyA, an antibody recognition sequence, and a library amplification sequence, the antibody-conjugated nucleic acid PolyA and transcriptome PolyA are captured at the same time with the PolyT structure on the chip, such that simultaneous construction of the proteome library and the transcriptome library, as well as subsequent co-localization detection of the proteome and transcriptome, are implemented, thereby realizing full-tissue field-of-view detection of a transcriptome and a proteome.

In a preferred embodiment, S1 comprises: S11, the chip is blocked (to reduce the occurrence of non-specific hybridization) by using a blocking solution to obtain a blocked chip; S12, the nucleic acid-conjugated antibody and the blocked chip are incubated to obtain a protein capture chip; and S13, a permeabilization treatment is performed on the protein capture chip to capture the mRNA, so as to obtain a permeabilized chip.

As described above, in the nucleic acid sequence on the nucleic acid-conjugated antibody, the PCR adapter region is configured to achieve subsequent library amplification. **The** antibody barcode region is configured to identify antibody types. **The** same type of antibody has the same barcode sequence. **The** PolyA sequence may be hybridized with the PolyT of a probe on the chip. **The** antibody portion of the nucleic acid-conjugated antibody still has biological activity and thus can be bound to a corresponding antigenic epitope on the sectioned sample. In the above preferred embodiments, in order to prevent a conjugated nucleic acid from being hybridized with the probe on the chip to trigger a non-specific signal as far as possible, the chip is blocked with the blocking solution before antibody incubation.

In a preferred embodiment, the blocking solution comprises at final concentrations of:
3×SSC, 5%-10%(v/v) serum (which may be, for example, 5%, 6%, 7%, 8%, 9%, or 10%), 0.1%(v/v) Triton-x-100, 0.1 mg/mL-1 mg/mL salmon sperm DNA (which may be, for example, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1 mg/mL), 0.5 µM-10 µM 22 bp-32 bp PolyA (which may be, for example, 0.5 µM, 0.6 µM, 0.7 µM, 0.8 µM, 0.9 µM, 1.0 µM, 1.5 µM, 2.0 µM, 2.5 µM, 3.0 µM, 3.5 µM, 4.0 µM, 4.5 µM, 5.0 µM, 5.5 µM, 6.0 µM, 6.5 µM, 7.0 µM, 7.5 µM, 8.0 µM, 8.5 µM, 9.0 µM, 9.5 µM, 10.0 µM), and 5%(v/v) RNase inhibitor. Preferably, a concentration of the 22 bp-32 bp PolyA in the blocking solution is 1 µM-10 µM, more preferably 5 µM-10 µM, and further preferably 8 µM-10 µM; and preferably, a concentration of the salmon sperm DNA in the blocking solution is 0.5 mg/mL-1 mg/mL.

In the above preferred embodiments, a pH value of the blocking solution is preferably 6-9. In the above preferred embodiments, the blocking solution at a salt ion concentration from 1× to 7× is suitable and has no significant adverse effects on transcriptome capture.

In the present application, through tests, the inventors further discovered that: 1) a blocking effect of the blocking solution containing 10 µM 22-32 bp PolyA is superior to that of the solution containing 0.5 µM 22-32 bp PolyA. 2) An effect of the blocking solution containing 0.1 mg/ml-1 mg/ml DNA (for example, the salmon sperm DNA) is better than that of the solution without adding the DNA.

The inventors also discovered that: 3) direct use of the blocking solution or PBS containing 0.5 uM-10 uM PolyA (22-32 bp) and 0.1-1 mg/ml DNA (the salmon sperm DNA), or 2-3×SSC buffer containing 0.5 uM-10 uM PolyA (22-32 bp) and 0.1-1 mg/ml DNA (the salmon sperm DNA) as an antibody diluent is superior to direct use of PBS or 2-3×SSC as an antibody diluent. 4) When the blocking solution and the antibody diluent do not contain PolyA (22-32 bp), experiments are extremely easy to fail, and the background is too high. 5) The absence of the PolyA (22-32 bp) in the antibody diluent easily causes unstable results with background higher than the signals.

Therefore, in a preferred embodiment, in S12, before incubating the nucleic acid-conjugated antibody with the blocked chip, the method further comprises a step of diluting the nucleic acid-conjugated antibody with an antibody diluent. The antibody diluent is any one selected from the group consisting of: a blocking solution, a PBS buffer containing PolyA and DNA, or 2-3×SSC buffer containing PolyA and DNA. In the PBS buffer and the SSC buffer, a concentration of the PolyA is 0.5 µM-10 µM, a concentration of the DNA is 0.1 mg/ml-1 mg/ml, and preferably, the DNA is salmon sperm DNA.

In a preferred embodiment, in S12, the nucleic acid-conjugated antibody and the blocked chip are incubated to bind the nucleic acid-conjugated antibody to an antigen, and then an antibody-antigen complex is obtained; and the antibody-antigen complex is placed at 60 °C-70 °C (which may be, for example, 60 °C, 61 °C, 62 °C, 63 °C, 64 °C, 65 °C, 66 °C, 67 °C, 68 °C, 69 °C, or 70 °C), preferably 65 °C-70 °C, to react in 0.1×SSC buffer for 5-15 min, so as to remove non-specific binding of the nucleic acid-conjugated antibody to the tissue or the chip, preferably for 10 min, thereby obtaining the protein capture chip.

In the above preferred embodiments, in order to prevent the conjugated nucleic acid from being hybridized with the probe on the chip to trigger a non-specific signal as far as possible, by optimizing the blocking solution or performing the reaction in the 0.1×SSC buffer (it is to be noted that, the 0.1×SSC buffer here is not the only feasible one and not for limitations, but rather exemplary, for example, a 3×SSC buffer also has the similar effect) for 10 min at 60 °C-70 °C after antibody-antigen binding, protein expression can be detected without affecting a capture level of the transcriptome. The formula optimization of the blocking solution is mainly reflected in the use of the salmon sperm DNA to block nucleic acid adsorption sites on the tissue, and the use of the PolyA in the blocking solution to block the non-specific signal from the probe outside the tissue that is hybridized with antibody-conjugated PolyA. The non-specific signal from the probe that is hybridized with the antibody-conjugated PolyA due to non-fully blocking can be removed by means of high temperature and low salt. Then, through an antibody antigen-specific binding reaction, an antibody-conjugated nucleic acid sequence portion is hybridized with PolyT of a probe near the antigen. Meanwhile, the mRNA in the tissue can also be hybridized with PolyT of a probe.

In a preferred embodiment, S2 comprises: reverse transcription on the captured mRNA and complementary strand synthesis on the nucleic acid sequence of the nucleic acid-conjugated antibody bound to the captured antigen protein are performed on the permeabilized chip, thereby obtaining a synthesized product, the synthesized product herein comprises the cDNA and the complementary strand of the nucleic acid sequence of the nucleic acid-conjugated antibody; and preferably, a temperature for the reverse transcription is 40 °C-44 °C, and a time is 1.5 hours-3 hours.

In a preferred embodiment, S3 comprises: at S31, tissue removal and probe digestion are successively performed on the sectioned sample after reverse transcription and complementary strand synthesis are performed on the permeabilized chip, so as to obtain a digestion solution containing the synthesized product; and at S32, the synthesized product is separated and purified from the digestion solution, and the proteome library and the transcriptome library are respectively obtained through amplification.

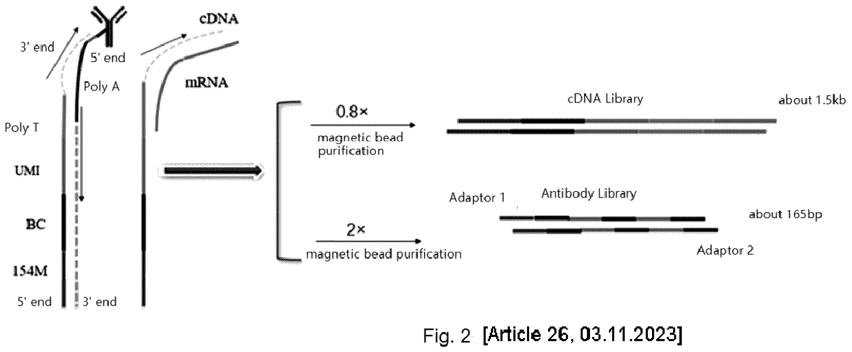

As shown in Fig. 2, through the reverse transcription reaction, the PolyT probe extends to incorporate an antibody barcode and a PCR adapter sequence, and at the same time, the PolyT probe hybridized with the mRNA are also extended to generate a cDNA sequence. This enables the PolyT probe to simultaneously carry type information of the antibody, mRNA information, and position information on the chip. Furthermore, since a transcriptome library fragment is about 1.5 kb, and a proteome library fragment is about 165 bp, separation of libraries containing different fragments is achieved by different amounts of magnetic beads, for example, the transcriptome is enriched and purified with 0.8× magnetic beads, the proteome is purified with 2× magnetic beads, and the transcriptome and proteome libraries can be separated by a method of differential enrichment and purification using the magnetic beads.

A tissue permeabilization solution used in the tissue permeabilization treatment step may be an existing permeabilization solution that can achieve a tissue section permeabilization effect. In a preferred embodiment, the tissue permeabilization solution is used for the permeabilization treatment, preferably, a temperature for the permeabilization treatment is 35 °C-38 °C, and a time for the permeabilization treatment is 3-20 min.

In a preferred embodiment, tissue removal is performed with a tissue removal solution, and digestion is performed with a digestion solution. Preferably, a temperature for tissue removal is 50 °C-60 °C, and a time for tissue removal is 20-40 min; and preferably, a temperature for digestion is 50 °C-60 °C, and a time for digestion is 2.5-4 hours.

It is to be noted that, the tissue removal solution, the digestion solution, and the permeabilization solution may all utilize corresponding products in existing commercially available kits. For example, spatiotemporal transcriptome kit of BGI: STOmics gene expression reagent kit-S1 (Cat. No.: 1000028496), where the tissue removal solution is TR buffer, the digestion solution is cDNA release buffer, and the permeabilization solution is PR Enzyme.

In a preferred embodiment, the Poly T is arranged as a plurality of clusters on the chip, and a distance between two adjacent clusters is 495-505 nm; preferably, in the nucleic acid-conjugated antibody, a length of the PCR adapter region is 18-23 bp, a length of the antibody barcode region is 8-20 bp, and a length of the PolyA is 22-35 bp. It is to be noted that, the nucleic acid-conjugated antibody in the present application is not specifically limited, and may be rationally selected according to research objectives, for example, may be one or more antibodies selected from the group consisting of: a CD169 antibody, a CD3 antibody, a CD8 antibody, a CD19 antibody, a CD20 antibody, a CD21 antibody, a CD45R-B220 antibody, a CD163 antibody, a CD38 antibody, a CD11b antibody, a CD140a antibody, a CD335 antibody, a CD371 antibody, a CD90.2 antibody, a TER-119 antibody, a CD274 antibody, a CD279 antibody, a CD56 antibody, a CD14 antibody, a CD340 antibody, a CD324 antibody, a CD38 antibody, a CD29 antibody, a CD68 antibody, a CD44 antibody, a CD21/35 antibody, an IgD antibody, a CD5 antibody, a CD4 antibody, an IgM antibody, a CD79b antibody, a CD11c antibody, an F4/80 antibody, a CD27 antibody, a CD31 antibody, or a CD8a antibody.

In the above preferred embodiments, the spacing between two Poly T cluster probes on the chip is set to about 500 nm, so as to realize co-localization detection of a whole transcriptome and a proteome at a resolution of 0.5 µm. Since a diameter of a single cell is generally about 10 µm, the chip of the present application can realize multi-omics detection at a single-cell resolution, and the protein detection effect is close to that of IF (gold standard, resolution of 0.2 µm).

A second typical implementation of the present application provides a multi-omics detection method. The method comprises: a proteome library and a transcriptome library of a sectioned sample to be detected are constructed with any one of the foregoing multi-omics library construction methods; and sequencing is performed on the proteome library and the transcriptome library to obtain proteome information and transcriptome information of the sectioned sample.

Applying the multi-omics detection method of the present application, the co-localization detection of the proteome and the transcriptome can be realized, full field-of-view detection on a single-cell scale can also be realized. Furthermore, 30 or more proteins can be detected at the same time.

A third typical implementation of the present application provides a multi-omics library construction kit. The kit comprises any of the following: a Poly A capture chip, a nucleic acid-conjugated antibody, a blocking solution, a tissue permeabilization solution, a tissue removal solution, and a digestion solution, and the kit must contain the blocking solution. The blocking solution comprises 3×SSC 5%-10%(v/v) of serum, 0.1%(v/v) of Triton-x-100, 0.1 mg/mL-1 mg/mL of salmon sperm DNA, 0.5 µM-10 µM of 22 bp-32 bp PolyA, and 5%(v/v) of a RNase inhibitor.

In some preferred embodiments, a concentration of the 22 bp-32 bp PolyA in the blocking solution is 1 µM-10 µM, more preferably 5 µM-10 µM, and further preferably 8 µM-10 µM; preferably, a concentration of the salmon sperm DNA in the blocking solution is 0.5 mg/mL-1 mg/mL; preferably, the Poly A capture chip refers to a capture chip containing Poly T, the nucleic acid-conjugated antibody comprises an antibody and a nucleic acid sequence coupled to the antibody, and the nucleic acid sequence successively comprises according to a direction away from the antibody: a PCR adapter region, an antibody barcode region, and a Poly A sequence; preferably, a plurality of clusters of Poly T on a Poly A capture chip are provided, and a distance between two adjacent clusters is 495 nm-505 nm; and preferably, in the nucleic acid-conjugated antibody, a length of the PCR adapter region is 18-23 bp, a length of the antibody barcode region is 8-20 bp, and a length of the PolyA is 22 bp-35 bp.

In a preferred embodiment, the multi-omics library construction method, detection method, and formulas of kit are as follows, respectively.
(1) A tissue section (10 µm) is frozen, and the tissue is dried, and then fixed for 30 min with -20 °C pre-cooled methanol, or fixed for 10 min with 4% PFA at room temperature.
(2) 100 µl of ssDNA dye is added for staining (a nucleic acid dye for staining cell nuclei, or DAPI or other dyes for staining may be used), a reaction is performed for 5 min at room temperature, after washing, the liquid is discarded, and washing is performed for three times with 0.1×SSC.
(3) 5 µl of glycerol is used for chip mounting, photographing is performed to collect fluorescence images, and soaking is performed for 5 min in 500 µl of 5×SSC with a 24-well plate.
   Note: (2) and (3) are only steps of staining the cell nuclei, and the staining of the cell nuclei is not a necessary step for library construction.
(4) A chip is taken out, 50 µl of washing buffer is added, the reaction is performed for 1 min, the liquid is discarded, this step is repeated, and the washing buffer is used for three times in total.
(5) 50 µl of the blocking solution is added, the reaction is performed for 30 min, the Poly A in the blocking solution may block a probe (removing a hybridization background) in non-tissue region of the chip, and the serum and the salmon sperm DNA may block non-specific adsorption sites from conjugated antibody on the tissue.
(6) A pre-prepared antibody is added for reaction for 45 min at room temperature, allowing the antibody to fully bind to an antigen.
(7) The antibody is washed and then discarded, washing is performed with 0.1×SSC, the reaction is performed for 1 min, washing is repeated for 5 times, and the tissue is air-dried.
(8) At 70 °C, the reaction is performed for 10 min in 0.1×SSC, and then washing is performed for 3 times with 0.1×SSC.
(9) Permeabilization is performed for a proper time with a tissue permeabilization solution, and is generally performed for 12 min at 37 °C. Then RT mix is added for reaction for 2 hours at 42 °C.
(10) 400 µl of the tissue removal solution is added, tissue digestion is performed for 30 min at 55 °C, the tissue removal solution is removed, and 400 µl of the digestion solution is added for digestion for 3 hours at 55 °C.
(11) cDNA is purified from the digestion solution, the proteome library and the transcriptome library are respectively amplified, DNA Nano-Balls (DNB) are prepared, and sequencing is performed on a machine.

The transcriptome library and the proteome library are prepared by a DNB library preparation method of a BGI sequencing platform and sequenced on MGI2000. The proteome library is sequenced with Read1 50 bp and Read2 15 bp; and the transcriptome library is sequenced with Read1 50 bp and Read2 50 bp. Data analysis is performed through a bioinformatics analysis pipeline to acquire specific information, so as to reconstruct the transcriptomic and proteomic profiles to their specific locations on the chip.

The beneficial effects of the present application are further described below with reference to specific examples.

### Example I

I. Experimental tissue: 10-µm frozen sections of mouse spleen tissue.
II. Reagent formula:
1) Blocking solution:

**Table 1:**

| Component | Manufacturer | Cat. No. | Single reaction system | Final concentration |
|---|---|---|---|---|
| SSC | Thermo | 15557036 | 7.5µl | 3×SSC |
| Horse serum | Thermo | 26050088 | 5 µl | 10% (v/v) |
| Triton-x-100 | Sigma | 93443 | 0.5µl | 0.1% (v/v) |
| Sheared salmon sperm DNA | Thermo | AM9680 | 5 µl | 1 mg/ml |
| 32 bp PolyA | BGI Write | / | 5 µl | 10 µM |
| RNase inhibitor | BGI | / | 2.5 µl | 5% (v/v) |
| Nuclease-free water | / | / | 24.5 µl | |
| Total | / | / | 50 µl | |

2) Antibody diluent: same as the blocking solution.
3) Washing Buffer (WB):

**Table 2:**

| Component | Reaction system per ml | Final concentration |
|---|---|---|
| SSC | 150 µl | 3×SSC |
| Triton-x-100 | 10 µl | 0.1% (v/v) |
| RNase inhibitor | 50 µl | 5% (v/v) |

4) Primary antibody incubation solution: the antibody diluent was used to dilute an antibody, the antibody was CDI69 antibody (Biolegend, Cat. No.: 142425), with a use amount of 0.5 µl/50 µl reaction system and with a final concentration of 0.25 µg/µl.
5) Secondary antibody incubation solution: 0.1 µl Goat anti-Rat 555/50 µl of the reaction system, and a final concentration of 0.4 µg/µl.
III. Experimental procedure:
1) The mouse spleen tissue was taken, and the tissue section (10 µm) was frozen and fixed for 30 min with pre-cooled methanol (-20 °C).
2) A chip was taken out, after the methanol volatilized and the tissue dried, 50µl of the WB was added to each chip for reaction for 1 min.
3) Liquid was discarded through suction, step 2) was repeated twice, and washing was performed for 3 times in total.
4) 50µl of the blocking solution was added to each chip for reaction for 30 min at room temperature.
5) The liquid was discarded through suction, 50 µl of the primary antibody incubation solution was added for reaction for 45 min at room temperature.
6) The reaction liquid was discarded through suction, 50 µl of a washing solution was added for reaction for 1 min, the step was repeated, and washing was performed for 3 times in total.
7) The secondary antibody incubation solution was added for reaction for 30 min at room temperature.
8) The reaction liquid was discarded through suction, 50 µl of a washing solution was added for reaction for 3 min at room temperature.
9) The reaction liquid was discarded through suction, 50 µl of a washing solution was added for reaction for 1 min, the step was repeated, and washing was performed for 3 times in total. The chip was air-dried, after the tissue was dried, 3 µl of glycerol was added for chip mounting, and fluorescence images were collected.
   Note: the foregoing steps 1)-9) were experimental procedures for IF. The following steps were a protein capture step:
10) Glass coverslip was removed, the chip was placed in a 5×SSC solution for reaction for 5 min, and the glycerol was removed through washing.
11) The chip was put in a 24-well plate, 1 ml of 0.1×SSC was added for reaction for 10 min at 65 °C.
12) The chip was taken out, the chip was washed once with the 0.1×SSC, a permeabilization reagent was added for reaction for 12 min at 37 °C.
13) The chip was washed once with 0.1×SSC, RT mix was added for reaction for 2 hours at 42 °C.
14) 400 µl of a tissue removal solution was added for digestion for 30 min at 55 °C, the tissue removal solution was removed, and 400 µl of a digestion solution was added for digestion for 3 hours at 55 °C.
15) cDNA was purified from the digestion solution, a proteome library and a transcriptome library were respectively amplified, DNBs were prepared, and sequencing was performed on a machine.
16) Transcriptome sequencing used a sequencing solution of 50 bp Read1+ 50 bp Read2; and proteome sequencing used a sequencing solution of 50 bp Read1+ 15 bp Read2.
17) Visualization treatment on sequencing results: data analysis might be performed through an existing bioinformatics analysis pipeline to acquire specific information, so as to reconstruct the transcriptomic and proteomic profile to their specific locations on the chip.

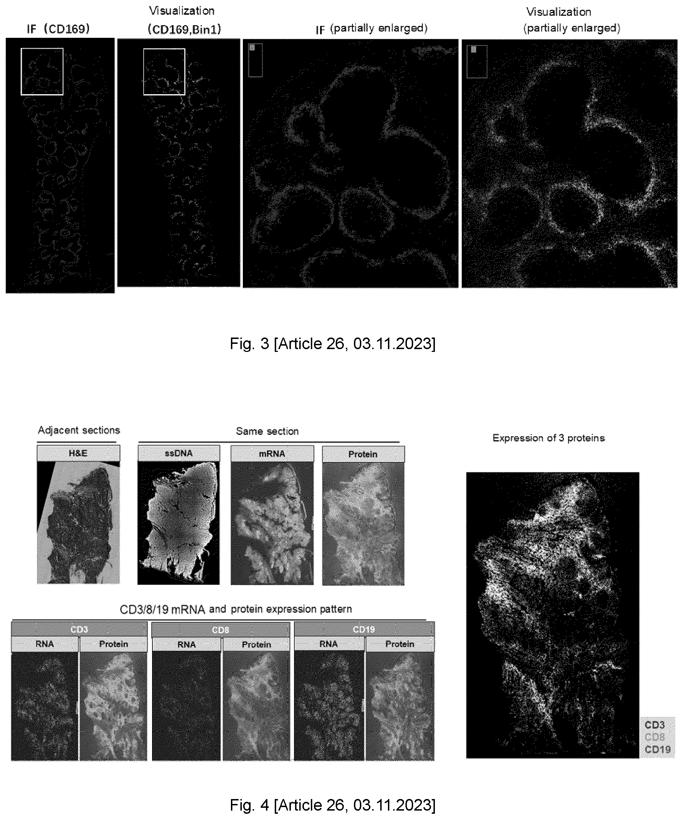

IV. Experimental results and analysis:
As shown in Fig. 3, visualization results of the CD169 had high similarity to corresponding IF. From a partially enlarged view on the right side, it might be seen that visualization was almost identical to the **IF,** and an IF effect could be achieved.

### Example II

I. Experimental tissue: 10-µm frozen sections of human tonsil tissue.
II. Reagent formula:
   1) Blocking solution: the same as Table 1 in Example I.
   2) Antibody diluent: the same as the blocking solution.
   3) WB: the same as Table 2 in Example I.
   4) Primary antibody incubation solution: the antibody diluent was used to dilute an antibody, with a use amount of 0.5 µl/50 µl of the reaction system and with a final concentration of 0.25 µg/µl. The antibody used a CD3 antibody (Biolegend, Cat. No.: 300475); CD8 antibody (Biolegend, Cat. No.: 344751); and CD19 antibody (Biolegend, Cat. No.: 302259).
   5) Secondary antibody incubation solution: 0.1 µl Goat anti-Rat 555/50 µl of the reaction system, and a final concentration of 0.4 µg/µl.
III. Experimental procedure:
   1) The human tonsil tissue was taken, and the tissue section (10 µm) was frozen and fixed for 30 min with pre-cooled methanol (-20 °C).
   2) 100 µl of DAPI was added for nuclear staining, the reaction was performed for 5 min at room temperature, the liquid was discarded through suction, and washing was performed for 3 times with 0.1×SSC.
   3) 5 µl of glycerol was used for chip mounting, photographing was performed to collect fluorescence images, and soaking was performed for 5 min in 500 µl of 5×SSC with a 24-well plate.
   4) A chip was taken out, 50 µl of WB was added for reaction for 1 min, the liquid was discarded through suction, this step was repeated, and the WB was used for three times in total.
   5) 50 µl of the blocking solution was added for reaction for 30 min at room temperature.
   6) A pre-prepared primary antibody incubation solution was added for reaction for 45 min at room temperature, causing the antibody to fully bind to an antigen.
   7) The antibody was discarded through suction, washing was performed with 0.1×SSC, the reaction was performed for 1 min, washing was repeated for 5 times, and the tissue was air-dried.
   8) At 70 °C, the reaction was performed for 10 min in 3×SSC, and then washing was performed for 3 times with 0.1×SSC.
   9) Permeabilization was performed for a proper time with the tissue permeabilization solution and was generally performed for 12 min at 37 °C. Then RT mix is added for reaction for 2 hours at 42 °C.
   10) 400 µl of a tissue removal solution was added for digestion for 30 min at 55 °C, the tissue removal solution was removed, and 400 µl of a digestion solution was added for digestion for 3 hours at 55 °C.
   11) cDNA was purified from the digestion solution, the proteome library and the transcriptome library were respectively amplified, DNBs were prepared, and sequencing was performed on a machine.
   12) The transcriptome library and the proteome library were prepared as a DNB library preparation method of a BGI sequencing platform and sequenced on MGI2000. The proteome library was sequenced with Read1 50 bp and Read2 15 bp; and the transcriptome library was sequenced with Read1 50 bp and Read2 50 bp. Data analysis was performed through a bioinformatics analysis pipeline to acquire specific information, so as to reconstruct the transcriptomic and proteomic profile to their specific locations on the chip.

### IV. Result and analysis

As shown in Fig. **4****,** protein expression of CD3/8/19 exhibited a spatially concordant pattern with the transcriptomic expression, and the co-localization detection of both the transcriptome and the proteome within a single tissue section was realized.

### Example III

I. Experimental tissue: mouse spleen tissue.
II. Reagent formula:
   1) Blocking solution: the same as Table 1 in Example I.
   2) Antibody diluent: the same as the blocking solution.
   3) WB: the same as Table 2 in Example I.
   4) Primary antibody incubation solution: the antibody diluent was used to dilute an antibody, with a use amount of 0.5 µl/50 µl of the reaction system and with a final concentration of 0.25 µg/µl. The antibody used a CD29 antibody (Biolegend, Cat. No.: 102233); CD68 antibody (Biolegend, Cat. No.: 137031); CD44 antibody (Biolegend, Cat. No.: 103045); CD21/35 antibody (Biolegend, Cat. No.: 123427); IgD antibody (Biolegend, Cat. No.: 405745); CD5 antibody (Biolegend, Cat. No.: 100637); CD4 antibody (Biolegend, Cat. No.: 100569); IgM antibody (Biolegend, Cat. No.: 406535); CD79b antibody (Biolegend, Cat. No.: 132811); CD169 antibody (Biolegend, Cat. No.: 142425); CD11c antibody (Biolegend, Cat. No.: 117355); F4/80 antibody (Biolegend, Cat. No.: 123153); CD27 antibody (Biolegend, Cat. No.: 124235); CD31 antibody (Biolegend, Cat. No.: 102437); and CD8a antibody (Biolegend, Cat. No.: 100773).
III. Experimental procedure: experimental procedures the same as Example II were used, and a tissue type was changed to the mouse spleen tissue.
IV. Result and analysis

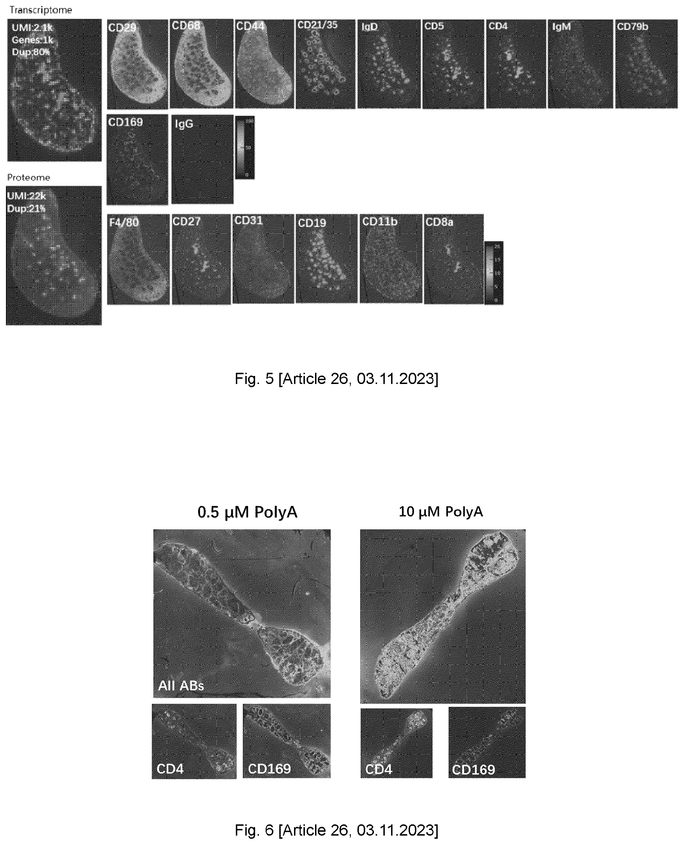

As shown in Fig. 5, expression of 16-proteins+transcriptome was successfully detected in the mouse spleen tissue.

### Example IV

I. Experimental tissue: mouse spleen tissue.
II. Reagent formula:
   1) Blocking solution: in two groups of experiments, concentrations of the PolyA in the blocking solution were respectively 0.5 µM and 10 µM, and other components in the formula of the blocking solution were the same as Table 1 in Example I.
   2) Antibody diluent: the same as the blocking solution.
   3) WB: the same as Table 2 in Example I.
   4) Primary antibody incubation solution: the antibody diluent was used to dilute an antibody, with a use amount of 0.5 µl/50 µl of the reaction system and with a final concentration of 0.25 µg/µl. The antibody used a CD4 antibody (Biolegend, Cat. No.: 100569); and CD169 antibody (Biolegend, Cat. No.: 142425).
III. Experimental procedure:

The experimental procedures in Example II were used, the tissue type was changed to the mouse spleen tissue, and the sequencing solution of 50-bp Read1 + 15-bp Read2 was used to separately sequence the proteome.

### IV. Result and analysis

As shown in Fig. 6, the proteome could be sequenced when the blocking solution contained 10 µM or 0.5 µM PolyA. The blocking solution containing 10 µM PolyA was superior in blocking effect to the blocking solution containing 0.5 µM PolyA. The blocking solution had a dose effect.

### Example V

I. Experimental tissue: mouse spleen tissue.
II. Reagent formula:

A formula in the technical solution was used, and there were three experimental groups.
1) Blocking solution: the same as Table 1 in Example I.
2) Antibody diluent: a formula of a primary antibody incubation solution in a first group had no salmon sperm DNA or polyA; a primary antibody incubation solution in a second group only contained salmon sperm DNA; a primary antibody incubation solution in a third group only contained PolyA group; and a primary antibody incubation solution in a fourth group contained salmon sperm DNA and polyA. Specific component compositions were shown in Table 3.

**Table 3**

| | No salmon sperm DNA or polyA group | Only containing salmon sperm DNA group | Only containing PolyA group | Containing salmon sperm DNA and polyA group |
|---|---|---|---|---|
| SSC | 3×SSC | 3×SSC | 3×SSC | 3×SSC |
| Horse serum | 10% (v/v) | 10% (v/v) | 10% (v/v) | 10% (v/v) |
| Triton-x-100 | 0.1% (v/v) | 0.1% (v/v) | 0.1% (v/v) | 0.1% (v/v) |
| Sheared salmon sperm DNA | None | 1 mg/ml | None | 1 mg/ml |
| 32 bp PolyA | None | None | 10 µM | 10 µM |
| RNase inhibitor | 5% (v/v) | 5% (v/v) | 5% (v/v) | 5% (v/v) |
| Nuclease-free water | / | / | / | / |
| Total | 50 µl | 50 µl | 50 µl | 50 µl |

3) WB: the same as Table 2 in Example I.
4) Primary antibody incubation solution: corresponding antibody diluents were used to dilute an antibody in different experimental groups, with a use amount of 0.5 µl/50 µl of the reaction system and with a final concentration of 0.25 µg/µl. The antibody used CD4 antibody (Biolegend, Cat. No.: 100569); and CD45R antibody (Biolegend, Cat. No.: 103263).

### III. Experimental procedure:

Experimental procedures 1)-9) in Example I were used, the corresponding antibody diluents were used to dilute the antibody in different experimental groups, and experimental results were determined with an immunofluorescence signal-to-noise ratio.

### IV. Result and analysis

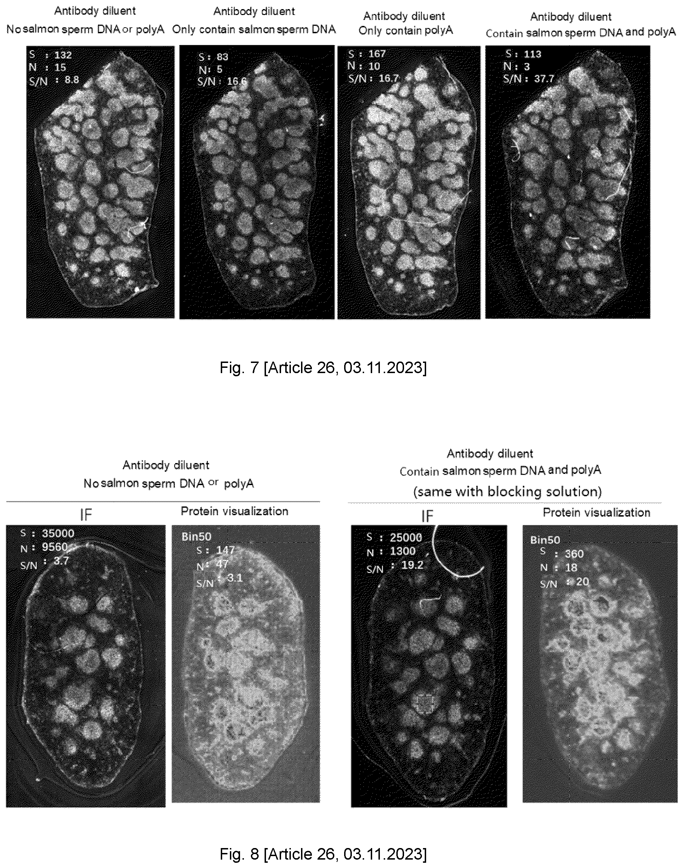

As shown in Fig. 7, a signal-to-noise ratio of the group where 10µM PolyA was added to the antibody diluent was 16.6, and a signal-to-noise ratio of the 1 mg/ml salmon sperm group was 16.7, which was significantly superior to a signal-to-noise ratio 8.8 of the no salmon sperm DNA or polyA group, indicating that the addition of the salmon sperm DNA and polyA might effectively increase the signal-to-noise ratio for detection. Furthermore, a signal-to-noise ratio of the group where both PolyA and salmon sperm DNA were added was 37.7, which was higher than that of the above two which used the salmon sperm DNA or polyA separately, indicating that a better effect might be achieved by combining salmon sperm DNA and polyA in a primary antibody diluent.

### Example VI

I. Experimental tissue: mouse spleen tissue.
II. Reagent formula:
1) Blocking solution: the same as Table 1 in Example I.
2) Antibody diluent: a formula of a primary antibody incubation solution in a first group had no salmon sperm DNA or polyA; and a primary antibody incubation solution in a second group was the same as the blocking solution, and contained the salmon sperm DNA and polyA. Specific component compositions were shown in Table 4.

**Table 4**

| | No salmon sperm DNA or polyA group | Containing salmon sperm DNA and polyA group (same as blocking solution) |
|---|---|---|
| SSC | 3×SSC | 3×SSC |
| Horse serum | 10% (v/v) | 10% (v/v) |
| Triton-x-100 | 0.1% (v/v) | 0.1% (v/v) |
| Sheared salmon sperm DNA | None | 1 mg/ml |
| 32 bp PolyA | None | 10 µM |
| RNase inhibitor | 5% (v/v) | 5% (v/v) |
| Nuclease-free water | / | / |
| Total | 50 µl | 50 µl |

3) WB: the same as Table 2 in Example I.
4) Primary antibody incubation solution: corresponding antibody diluents were used to dilute an antibody in different experimental groups, with a use amount of 0.5 µl/50 µl of the reaction system and with a final concentration of 0.25 µg/µl. The antibody used CD4 antibody (Biolegend, Cat. No.: 100569); and CD45R antibody (Biolegend, Cat. No.: 103263).
III. Experimental procedure:
Experimental procedures in Example I were used, a signal-to-noise ratio was determined through immunofluorescence and visualization.
IV. Result and analysis
As shown in Fig. 8, a signal-to-noise ratio of the IF when the antibody diluent was the blocking solution was 19.2, which was significantly higher than a signal-to-noise ratio 3.7 of the antibody diluent without containing polyA or salmon sperm DNA. Protein visualization also proved that an effect was better when the antibody diluent and the blocking solution were the same, and proved that the signal-to-noise ratio of protein visualization was very close to the signal-to-noise ratio of the IF.

### Example VII

I. Experimental tissue: mouse spleen tissue.
II. Reagent formula:
1) Blocking solution: components of the blocking solution were shown in Table 5, and there was no PolyA in the blocking solution.

**Table 5**

| | Final concentration |
|---|---|
| SSC | 3×SSC |
| Horse serum | 10% (v/v) |
| Triton-x-100 | 0.1% (v/v) |
| Sheared salmon sperm DNA | 1 mg/ml |
| 32 bp PolyA | None |
| RNase inhibitor | 5% (v/v) |
| Nuclease-free water | / |
| Total | 50 µl |

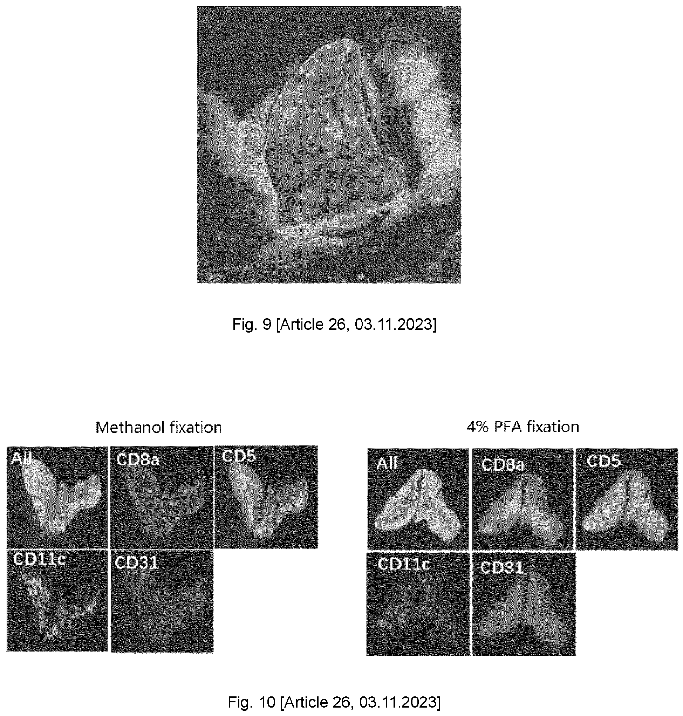

2) Antibody diluent: the same as the blocking solution.
3) WB: the same as Table 2 in Example I.
4) Primary antibody incubation solution: the antibody diluent was used to dilute an antibody, with a use amount of 0.5 µl/50 µl of the reaction system and with a final concentration of 0.25 µg/µl. The antibody used CD4 antibody (Biolegend, Cat. No.: 100569); and CD45R antibody (Biolegend, Cat. No.: 103263).
III. Experimental procedure: experimental procedures in Example II were used, and sequencing was separately performed on a proteome.
IV. Result and analysis: as shown in Fig. 9, the experiment failed when the blocking solution without containing PolyA, and signals were mostly concentrated outside the tissue.

### Example VIII

I. Experimental tissue: mouse thymus tissue.
II. Reagent formula:
   1) Blocking solution: the same as Table 1 in Example I.
   2) Antibody diluent: the same as the blocking solution.
   3) WB: the same as Table 2 in Example I.
   4) Primary antibody incubation solution: the corresponding antibody diluent was used to dilute an antibody, with a use amount of 0.5 µl/50 µl of the reaction system and with a final concentration of 0.25 µg/µl. The antibody used CD5 antibody (Biolegend, Cat. No.: 100637); CD8a antibody (Biolegend, Cat. No.: 100773); CD11c antibody (Biolegend, Cat. No.: 117355); and CD31 antibody (Biolegend, Cat. No.: 102437).
III. Experimental procedure:
   A group sample was fixed with PFA by referring to the following description, in steps 1)-2) of a methanol fixed group, fixation was performed for 30 min with pre-cooled methanol (-20 °C), and after the section was dried, the remaining steps were processed according to steps 3)-16).
   1) Tissue in the experimental group was taken, and the tissue section (10 µm) was frozen, dried for 3 min, and placed in 4%PFA for fixation for 10 min.
   2) 400 ul of the WB was added in a 24-well plate, the tissue was immediately taken out after being fixed, and in order to prevent the tissue from drying, the tissue was immediately added to the well plate for washing for twice.
   3) 50µl of the blocking solution was added to each chip for reaction for 30 min at room temperature.
   4) The liquid was discarded through suction, 50 µl of the primary antibody incubation solution was added for reaction for 45 min at room temperature.
   5) The reaction liquid was discarded through suction, 50 µl of a washing solution was added for reaction for 1 min, the step was repeated, and washing was performed for 3 times in total.
   6) The secondary antibody incubation solution was added for reaction for 30 min at room temperature.
   7) The reaction liquid was discarded through suction, 50 µl of a washing solution was added for reaction for 3 min at room temperature.
   8) The reaction liquid was discarded through suction, 50 µl of a washing solution was added for reaction for 1 min, the step was repeated, and washing was performed for 3 times in total. The chip was air-dried, after the tissue was dried, 3 µl of glycerol was added for chip mounting, and fluorescence images were collected.
      Note: the foregoing steps 1)-8) were experimental procedures for IF. The following were steps for a protein capture:
   9) Glass coverslip was removed, the chip was placed in a 5×SSC solution for reaction for 5 min, and the glycerol was removed through washing.
   10) The chip was put in the 24-well plate, 1 ml of 3×SSC was added for reaction for 10 min at 70 °C.
   11) The chip was taken out, the chip was washed once with 0.1×SSC, a permeabilization reagent was added for reaction for 12 min at 37 °C.
   12) The chip was washed once with 0.1×SSC, RT mix was added for reaction for 2 hours at 42 °C.
   13) 400 µl of a tissue removal solution was added for digestion for 30 min at 55 °C, the tissue removal solution was removed, and 400 µl of a digestion solution was added for digestion for 3 hours at 55 °C.
   14) cDNA was purified from the digestion solution, a proteome library and a transcriptome library were respectively amplified, DNBs were prepared, and sequencing was performed on a machine.
   15) Transcriptome sequencing used a sequencing solution of 50-bp Read1 + 50-bp Read2; and proteome sequencing used a sequencing solution of 50-bp Read1 + 15-bp Read2.
   16) Visualization treatment on sequencing results: data analysis might be performed through an existing bioinformatics analysis pipeline to acquire specific information, so as to reconstruct the transcriptomic and proteomic profile to their specific locations on the chip.
IV. Result and analysis
   As shown in Fig. 10, it was proved that the procedures adapted to methanol fixation and PFA fixation. However, it could also be noted that expression levels detected by different proteins were different, which might be caused by fixation differences of different fixation reagent on the antigens.

### Example IX

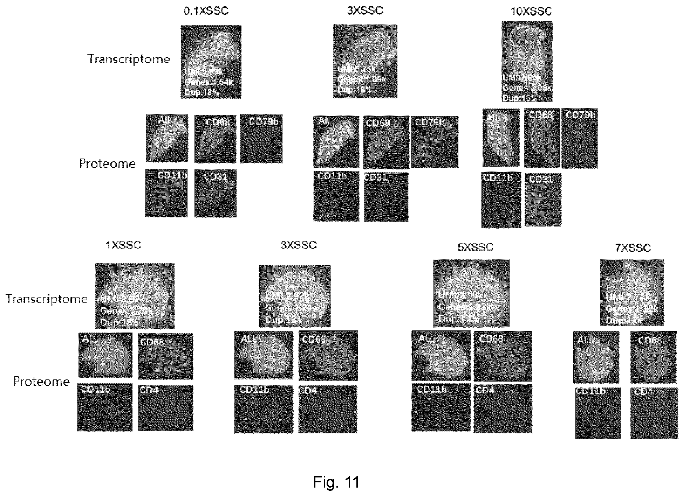

I. Experimental tissue: mouse liver tissue.
II. Reagent formula:
   1) Blocking solution: a formula was the same as Table 1 of the Example I, and SSC concentrations of 0.1×, 1×, 3×, 5×, 7×, and 10× were respectively used.
   2) Antibody diluent: the same as the blocking solution.
   3) WB: the same as Table 2 in Example I.
   4) Primary antibody incubation solution: the corresponding antibody diluent was used to dilute an antibody, with a use amount of 0.2 µl/50 µl of the reaction system and with a final concentration of 0.1 µg/µl. The antibody used CD68 antibody (Biolegend, Cat. No.: 137031); CD4 antibody (Biolegend, Cat. No.: 100569); CD79b antibody (Biolegend, Cat. No.: 132811); CD31 antibody (Biolegend, Cat. No.: 102437); and CD11b antibody (Biolegend, Cat. No.: 101265).
III. Experimental procedure:
   Referring to Example VIII, a PFA fixation sample was operated.
IV. Result and analysis
   As shown in Fig. 11, a tissue signal background was increased to a certain extent when an ion concentration of the blocking solution/antibody incubation solution was too high (10×) or too low (0.1×), for example, CD31 or CD11b. However, a salt ion concentration of the blocking solution between 1× and 7× has no significant difference and has no significant adverse effects on transcriptome capture.

### Example X

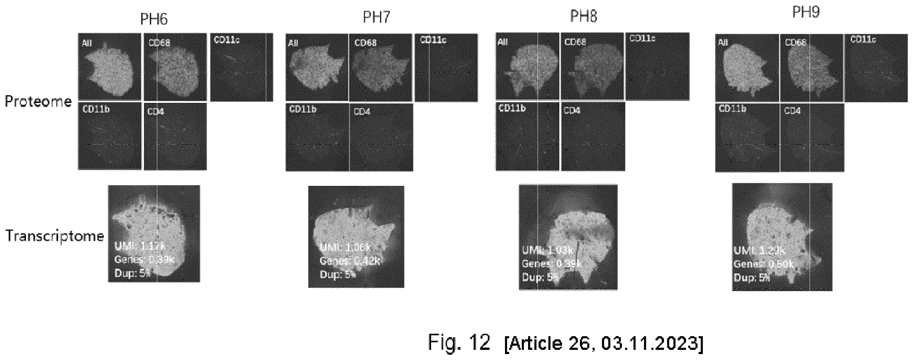

I. Experimental tissue: mouse liver tissue.
II. Reagent formula:
   1) Blocking solution: a formula was the same as Table 1 of the Example I, and the pH of a buffer was regulated to pH = 6, 7, 8, and 9.
   2) Antibody diluent: the same as the blocking solution.
   3) WB: the same as Table 2 in Example I.
   4) Primary antibody incubation solution: the corresponding antibody diluent was used to dilute an antibody, with a use amount of 0.2 µl/50 µl of the reaction system and with a final concentration of 0.1 µg/µl. The antibody used CD68 antibody (Biolegend, Cat. No.: 137031); CD4 antibody (Biolegend, Cat. No.: 100569); CD11b antibody (Biolegend, Cat. No.: 101265); and CD11c antibody (Biolegend, Cat. No.: 117355).
III. Experimental procedure:
   Referring to Example VIII, a PFA fixation sample was operated.
IV. Result and analysis
   As shown in Fig. 12, the pH value of the blocking solution between 6-9 had no significant difference on protein detection and had no significant impact on transcriptome capture.

### Example XI

I. Experimental tissue: mouse liver tissue.
II. Reagent formula:
   1) Blocking solution: a formula was the same as Table 1 in Example I.
   2) Antibody diluent: the same as the blocking solution.
   3) WB: the same as Table 2 in Example I.
   4) Primary antibody incubation solution: the corresponding antibody diluent was used to dilute an antibody, with a use amount of 0.2 µl/50 µl of the reaction system and with a final concentration of 0.1 µg/µl. The antibody used a CD68 antibody (Biolegend, Cat. No.: 137031); CD4 antibody (Biolegend, Cat. No.: 100569); CD11b antibody (Biolegend, Cat. No.: 101265); CD11c antibody (Biolegend, Cat. No.: 117355); and CD31 antibody (Biolegend, Cat. No.: 102437).
III. Experimental procedure:
   For a normal procedure group, referring to the experimental procedures in Example VIII, the PFA fixation sample was operated.

For a KOH elution group, when step13) in Example VIII was performed, the tissue digestion solution step was replaced with KOH elution, such that a procedure time was shortened. Specific operations were as follows.
1) After an RT reaction ended, the RT reaction solution was discarded through suction; and 100 µL of a 0.1xSSC solution was added to the chip for washing.
2) The 0.1XSSC was discarded through suction, 90 µL of 0.1M KOH was added to the chip, ensuring that the KOH uniformly covered the entire chip, and the chip was placed for 30 min at room temperature.
3) The KOH solution was sucked from a corner of the chip and transferred into a PCR tube, the PH value was regulated to 7.4, and proteome products and transcriptome products were respectively purified for PCR amplification.
4) A proteome library and a transcriptome library were respectively constructed, DNBs were prepared, and sequencing was performed on a machine.
5) Transcriptome sequencing used a sequencing solution of 50-bp Read1 + 50-bp Read2; and proteome sequencing used a sequencing solution of 50-bp Read1 + 15-bp Read2.
6) Visualization treatment on sequencing results: data analysis might be performed through an existing bioinformatics analysis pipeline to acquire specific information, so as to reconstruct the transcriptomic and proteomic profile to their specific locations on the chip.

### IV. Result and analysis

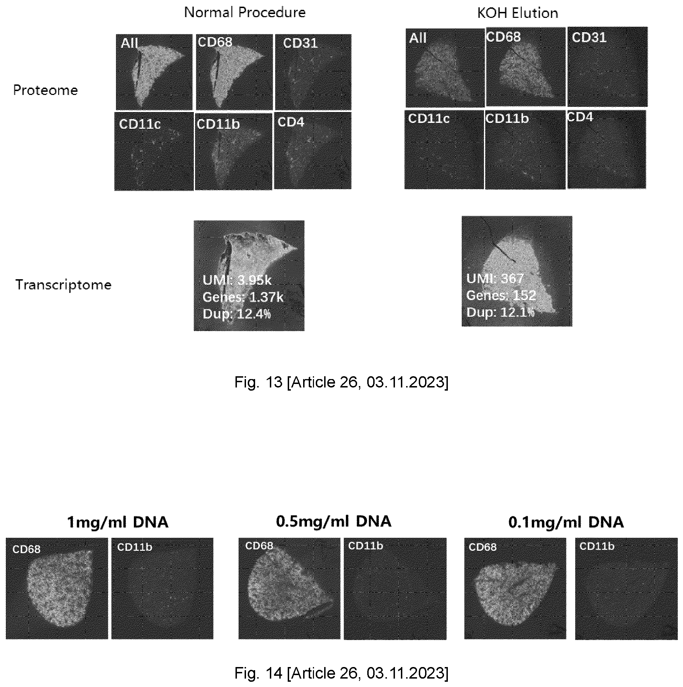

As shown in Fig. 13, the proteome and the transcriptome might also be successfully detected together by means of KOH elution. However, the KOH elution step needed to be further optimized to improve protein and transcription capture.

### Example XII

I. Experimental tissue: mouse liver tissue.
II. Reagent formula:
   1) Blocking solution: a formula was same as Table 1 in Example I, but concentrations of the salmon sperm DNA of 1 mg/ml, 0.5 mg/ml, and 0.1 mg/ml were used, respectively.
   2) Antibody diluent: the same as the blocking solution.
   3) WB: the same as Table 2 in Example I.
   4) Primary antibody incubation solution: the corresponding antibody diluent was used to dilute an antibody, with a use amount of 0.2 µl/50 µl of the reaction system and with a final concentration of 0.1 µg/µl. The antibody used CD68 antibody (Biolegend, Cat. No.: 137031); and CD11b antibody (Biolegend, Cat. No.: 101265).
III. Experimental procedure:
   For a normal procedure group, referring to the experimental procedures in Example VIII, the PFA fixation sample was operated.
IV. Result and analysis
   As shown in Fig. 14, the concentrations of the salmon sperm DNA between 0.1 mg/ml and 1 mg/ml could all achieve similar visualization effects.

From the above descriptions, it might be seen that the embodiments of the present invention achieved the following technical effects. According to the multi-omics library construction method and the multi-omics detection method of the present application, applying an antibody-conjugated nucleic acid having PolyA, an antibody recognition sequence, and a library amplification sequence, the antibody-conjugated nucleic acid PolyA and transcriptome PolyA were captured at the same time with the PolyT structure on the chip, such that simultaneous construction of the proteome library and the transcriptome library, as well as subsequent co-localization detection of the proteome and transcriptome, were implemented. The method did not require a laser to take pictures and only required simple devices.

Furthermore, in the solution of the present application, when the chip contained the Ploy T clusters with the spacing of 500 nm was used to capture the antibody containing PolyA and the mRNA, the non-specificity of antibody hybridization was reduced by optimizing the blocking solution and the antibody hybridization procedure, without affecting transcriptome capture and library construction, such that the co-localization detection of the transcriptome and the proteome on a single-cell scale under a full-tissue field of view was realized, and 30 or more proteins might be detected at the same time under the optimal situation.

The above are only the preferred embodiments of the present invention and are not intended to limit the present invention. For those skilled in the art, the present invention may have various modifications and variations. Any modifications, equivalent replacements, improvements and the like made within the spirit and principle of the present invention all fall within the scope of protection of the present invention.

## Claims

1. A multi-omics library construction method, comprising:
S1, placing a sectioned sample on a chip having a Poly A capture function to perform protein capture and mRNA capture, wherein
the protein capture is performed with a nucleic acid-conjugated antibody,
the nucleic acid-conjugated antibody comprises an antibody and a nucleic acid sequence conjugated thereto, the nucleic acid sequence successively comprises, in a direction away from the antibody: a PCR adapter region, an antibody barcode region, and a Poly A sequence,
the chip comprises Poly **T,**
the nucleic acid-conjugated antibody binds to the Poly T on the chip through the Poly A sequence to perform antibody-bound antigen protein capture, and
mRNA in the sectioned sample binds to the Poly T on the chip through the Poly A of the mRNA to perform the mRNA capture;
S2, performing reverse transcription on the captured mRNA and complementary strand synthesis on the nucleic acid sequence of the antigen-bound nucleic acid-conjugated antibody to respectively obtain cDNA and a complementary strand of the nucleic acid sequence on the nucleic acid-conjugated antibody; and
S3, constructing libraries from the cDNA and the complementary strand of the nucleic acid sequence of the nucleic acid-conjugated antibody, thereby obtaining a transcriptome library and a proteome library of the sectioned sample.

2. The method according to claim 1, wherein, the S1 comprises:
S11, blocking the chip using a blocking solution to obtain a blocked chip;
S12, incubating the nucleic acid-conjugated antibody with the blocked chip to obtain a protein capture chip; and
S13, performing a permeabilization treatment on the protein capture chip to capture the mRNA and the nucleic acid sequence, thereby obtaining a permeabilized chip.

3. The method according to claim 2, wherein, the S2 comprises:
performing reverse transcription on the captured mRNA and complementary strand synthesis on the nucleic acid sequence on the permeabilized chip, thereby obtaining a synthesized product, wherein the synthesized product comprises the cDNA and the complementary strand of the nucleic acid sequence; and
preferably, a temperature for the reverse transcription is 40 °C-44 °C, and a time is 1.5 hours-3 hours.

4. The method according to claim 3, wherein, the S3 comprises:
S31, performing tissue removal and probe digestion successively on the sectioned sample after reverse transcription and complementary strand synthesis on the permeabilized chip, thereby obtaining a digestion solution containing the synthesized product; and
S32, separating and purifying the synthesized product from the digestion solution, and respectively obtaining the proteome library and the transcriptome library through amplification.

5. The method according to any one of claims 2 to **4,** wherein the blocking solution
comprises 3×SSC, 5%-10%(v/v) of serum, 0.1%(v/v) of Triton-x-100, 0.1 mg/mL-1 mg/mL of salmon sperm DNA, 0.5 µM-10 µM of 22 bp-32 bp PolyA, and 5%(v/v) of a RNase inhibitor;
preferably, a concentration of the 22 bp-32 bp PolyA in the blocking solution is 1 µM-10 µM, more preferably 5 µM-10 µM, and further preferably 8 µM-10 µM; and
preferably, a concentration of the salmon sperm DNA in the blocking solution is 0.5 mg/mL-1 mg/mL.

6. The method according to claim 5, wherein in the S12, before incubating the nucleic
acid-conjugated antibody with the blocked chip, the method further comprises a step of diluting the nucleic acid-conjugated antibody with an antibody diluent, wherein
the antibody diluent is any one selected from the group consisting of a blocking solution, a PBS buffer containing PolyA and DNA, or 2-3×SSC buffer containing PolyA and DNA; in the PBS buffer and the SSC buffer, a concentration of the PolyA is 0.5 µM-10 µM, a concentration of the DNA is 0.1 mg/ml-1 mg/ml, and preferably, the DNA is salmon sperm DNA; and
preferably, the S12 comprises:
incubating the nucleic acid-conjugated antibody with the blocked chip to bind the nucleic acid-conjugated antibody to an antigen, thereby obtaining an antibody-antigen complex; and
placing the antibody-antigen complex at 60 °C-70 °C, preferably 65 °C-70 °C, to react in 0.1×SSC buffer for 5-15 min, preferably 10 min, thereby obtaining the protein capture chip.

7. The method according to claim 2 or 3, wherein a temperature for the permeabilization treatment is 35 °C-38 °C, and a time for the permeabilization treatment is 3 min-20 min.

8. The method according to claim **4,** wherein a temperature for the tissue removal is 50 °C-60 °C, and a time for the tissue removal is 20-40 min; and
preferably, a temperature for the digestion is 50 °C-60 °C, and a time for the digestion is 2.5 hours-4 hours.

9. The method according to claim 1, wherein the Poly T is arranged as a plurality of clusters on the chip, and a distance between two adjacent clusters is 495 nm-505 nm;
preferably, in the nucleic acid-conjugated antibody, a length of the PCR adapter region is 18 bp-23 bp, a length of the antibody barcode region is 8 bp-20 bp, and a length of the PolyA is 22 bp-35 bp; and
preferably, the antibody portion of the nucleic acid-conjugated antibody is one or more antibodies selected from the group consisting of: a CD169 antibody, a CD3 antibody, a CD8 antibody, a CD19 antibody, a CD20 antibody, a CD21 antibody, a CD45R-B220 antibody, a CD163 antibody, a CD38 antibody, a CD11b antibody, a CD140a antibody, a CD335 antibody, a CD371 antibody, a CD90.2 antibody, a TER-119 antibody, a CD274 antibody, a CD279 antibody, a CD56 antibody, a CD14 antibody, a CD340 antibody, a CD324 antibody, a CD38 antibody, a CD29 antibody, a CD68 antibody, a CD44 antibody, a CD21/35 antibody, an Ig D antibody, a CD5 antibody, a CD4 antibody, an IgM antibody, a CD79b antibody, a CD11c antibody, an F4/80 antibody, a CD27 antibody, a CD31 antibody, or a CD8a antibody.

10. A multi-omics detection method, comprising:
constructing a proteome library and a transcriptome library of a sectioned sample to be detected with the multi-omics library construction method according to any one of claims 1 to 9; and
performing sequencing on the proteome library and the transcriptome library to obtain proteome information and transcriptome information of the sectioned sample.

11. A kit for constructing multi-omics libraries, comprising any of the following: a Poly A
capture chip, a nucleic acid-conjugated antibody, a blocking solution, a tissue permeabilization solution, a tissue removal solution, and a digestion solution, wherein
the blocking solution comprises 3×SSC, 5%-10%(v/v) of serum, 0.1%(v/v) of Triton-x-100, 0.1 mg/mL-1 mg/mL of salmon sperm DNA, 0.5 µM-10 µM of 22 bp-32 bp PolyA, and 5%(v/v) of a RNase inhibitor.

12. The kit according to claim 11, wherein a concentration of the 22 bp-32 bp PolyA in
the blocking solution is 1 µM-10 µM, more preferably 5 µM-10 µM, and further preferably 8 µM-10 µM;
preferably, a concentration of the salmon sperm DNA in the blocking solution is 0.5 mg/mL-1 mg/mL;
preferably, the Poly A capture chip refers to a capture chip containing Poly **T,** the nucleic acid-conjugated antibody comprises an antibody and a nucleic acid sequence conjugated thereto, and the nucleic acid sequence successively comprises in a direction away from the antibody: a PCR adapter region, an antibody barcode region, and a Poly A sequence;
preferably, the Poly T is arranged as a plurality of clusters on the Poly A capture chip, and a distance between two adjacent clusters is 495 nm-505 nm; and
preferably, in the nucleic acid-conjugated antibody, a length of the PCR adapter region is 18-23 bp, a length of the antibody barcode region is 8-20 bp, and a length of the PolyA is 22 bp-35 bp.
